# EUROPÄISCHE PATENTSCHRIFT

(11) **EP 2 108 332 B1**
(45) Veröffentlichungstag und Bekanntmachung des Hinweises auf die Patenterteilung: **02.08.2017**
(21) Anmeldenummer: 09008102.7
(22) Anmeldetag: 15.08.2003
(51) Int. Cl.: A61C 8/00, A61B 17/68, A61L 27/50

(54) **Augmentationselement zur Implantation in Knochengewebe oder in durch Knochenersatzmaterial ergänztem Knochengewebe**
Augmentation element for implantation in bone tissue or bone tissue supplemented with bone replacement material
Element d'augmentation destiné à l'implantation dans du tissu osseux ou dans du tissu osseux remplacé par du matériau de remplacement d'os

(30) Priorität: 23.08.2002 CH 14522002
(43) Veröffentlichungstag der Anmeldung: 14.10.2009
(62) Teilanmeldung aus: 03792085.7
(73) Patentinhaber: Woodwelding AG, 6304 Zug (CH)
(72) Erfinder: Mayer, Jörg, 5702 Niederlenz (CH); Aeschlimann, Marcel, 2514 Ligerz (CH); Torriani, Laurent, 2516 Lamboing (CH)
(74) Vertreter: Frei Patent Attorneys

(56) Entgegenhaltungen:
- WO-A1-02/069817
- DE-A- 19 741 087
- SU-A1- 929 072
- US-A- 4 222 128
- US-A- 5 972 368
- US-A- 6 080 161

## Beschreibung

Die Erfindung liegt auf dem Gebiete der Medizinaltechnik und betrifft ein Augmentationselement gemäss dem ersten unabhängigen Patentanspruch. Das Augmentationselement wird insbesondere in menschlichem oder tierischem Knochengewebe implantiert, kann aber auch in durch Knochenersatzmaterial ergänztem Knochengewebe implantiert werden. Dokument US 6 080 161 A offenbart eine resorbierbare Osteosynthese-Platte mit Löchern und verflüssigbaren Verankerungselementen.

Dokument WO02069817 offenbart Implantate, die mindestens teilweise aus einem durch mechanische Energie verflüssigbaren Material bestehen. Das erfindungsgemäße Augmentationselement zur Implantation in menschlichem oder tierischem Knochengewebe oder durch Knochenersatzmaterial ergänztes Knochengewebe, weist einen zentralen Implantatteil aus einem porösen, das Knochenwachstum fördernden Material mit mindestens einer durchgehenden Öffnung und mindestens einem Stift aus einem durch mechanische Schwingungen verflüssigbaren Material auf, wobei der Stift durch die durchgehenden Öffnungen des zentralen Implantatteils führbar ist, wobei das zentrale Implantatteil in Kontakt mit dem Knochengewebe positionierbar und der mindestens eine in der durchgehenden Öffnung positionierte Stift durch eine auf den Stiftquerschnitt abgestimmte Sonotrode, mit welcher Schwingungsenergie in den mindestens einen Stift eingekoppelt wird, gegen das Knochengewebe pressbar ist, wodurch das verflüssigbare Material mindestens teilweise verflüssigt und in das Knochengewebe und den zentralen Implantatteil pressbar ist.ßß.

Als verflüssigbare Materialien eignen sich zum Beispiel resorbierbare Polymere beispielsweise auf Milch- und/oder Glykolsäurebasis (PLA, PLLA, PGA, PLGA etc.) oder Polyhydroxyalkanoate (PHA), Polycaprolactone (PCL), Polysacharide, Polydioxanone (PD) Polyanhydride, Polypeptide oder entsprechende Copolymere oder Mischpolymere oder die genannten Polymere als Komponente enthaltende Verbundwerkstoffe. Als nicht resorbierbare Polymere sind Thermoplasten wie beispielsweise Polyolefine (z.B. Polyethylen), Polyacrylate, Polymetacrylate, Polycarbonate, Polyamide, Polyester, Polyurethane, Polysulfone, Polyarylketone, Polyimide, Polyphenylsulfide oder Flüssig-Kristall-Polymere (liquid cristal polymers LCPs), Polyacetale, halogenierte Polymere, insbesondere halogenierte Polyolefine, Polyphenylensulfide, Polysulfone, Polyether oder entsprechende Copolymere und Mischpolymere oder die genannten Polymere als Komponente enthaltende Verbundwerkstoffe geeignet. Anwendbare thixotrope Systeme sind resorbierbare, teilresorbierbare oder nicht resorbierbare, polymere, keramische oder hydraulische Zemente (z.B. Norian® von Synthes oder Sulfix® von Centerpulse).

Das verflüssigbare Material kann für weitere Funktionen Fremdphasen oder weitere Stoffe enthalten. Insbesondere kann das verflüssigbare Material durch Beimischen von Fasern oder Whiskern (z.B. Calziumphosphat-Keramiken oder -Gläser) verstärkt sein (Verbundwerkstoff). Es kann auch in situ quellende oder lösliche (porenbildende) Bestandteile (z.B. Polyester, Polysaccharide, Hydrogele, Natriumphosphat) oder in situ freizusetzende Stoffe mit therapeutischer Wirkung beispielsweise zur Förderung von Heilung und Regeneration (z.B. Wachstumsfaktoren, Antibiotika, Entzündungshemmer oder Puffer wie Natriumphosphat gegen nachteilige Wirkungen sauren Abbaus) enthalten. Wenn das verflüssigbare Material resorbierbar ist, werden solche Stoffe verzögert freigesetzt.

Der Implantatteil, der nicht aus dem verflüssigbaren Material besteht, ist nicht resorbierbar, wenn das Implantat im Körper verbleiben oder chirurgisch entfernt werden soll. Er kann aber auch mindestens teilweise aus einem resorbierbaren Material bestehen, das nach der Implantation allmählich durch vitales Gewebe ersetzt wird.

Die Ausgestaltung des Implantates und die Wahl des verflüssigbaren Materials sind aufeinander abzustimmen, derart, dass die Festigkeit des Formschlusses der erwarteten Belastung genügen kann, und derart, dass die Verflüssigung eine verantwortbare, das heisst möglichst geringe Freisetzung von Wärme mit sich bringt. Wenn verflüssigbare Materialien mit einer relativ hohen Erweichungstemperatur verwendet werden, ist vorteilhafterweise dafür zu sorgen, dass das Implantat als Ganzes (inklusive verflüssigbares Material) die mechanischen Schwingungen im Sinne eines Resonators leitet, so dass das verflüssigbare Material nur sehr örtlich, z.B. nur im Bereiche von entsprechend vorgesehenen Energierichtungsgebern in den Oberflächenbereichen der zweiten Art verflüssigt wird. Auf diese Weise kann die freigesetzte Wärmemenge in einem akzeptablen Rahmen gehalten werden. Insbesondere bei Verwendung eines Materials mit einer relativ tiefen Erweichungstemperatur oder eines ohne Freisetzung von Wärme verflüssigbaren Materials (z.B. thixotrope Zemente) kann die Verflüssigung auch im Innern des verflüssigbaren Materials (durch starke Dämpfung der anregenden Schwingungen) oder an Berührungsstellen zwischen zentralem und peripherem Implantatteil erfolgen.

Die Wärmebelastung des Gewebes während der Implantation kann weiter reduziert werden, indem der zentrale Implantatteil Materialien mit einer hohen Wärmeleitfähigkeit und/oder einer hohen Wärmekapazität (z.B. Siliziumcarbid) aufweist und gegebenenfalls mit Kühlkanälen versehen ist, durch die ein Kühlmedium geleitet wird. Eine beispielhafte Ausführungsform des erfmdungsgemässen Implantates wird anhand der folgenden Figur detailliert beschrieben. Dabei zeigt:

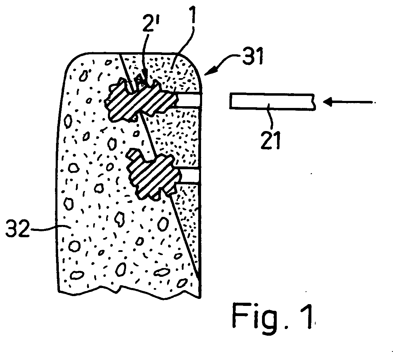

**Figur** 1 ein Augmentationselement als Beispiel eines erfindungsgemässen Implantates.
   Der zentrale Implantatteil 1 des Implantates gemäss Figur 1, der die Oberflächenbereiche 4 der ersten Art bildet, hat beispielsweise eine osseointegrative Funktion und besteht beispielsweise aus einem hochporösen Calziumphosphat, aus Knochenspan (patienteneigene Spongiosa) oder aus einem Gel. Es kann sich aber auch um eine Vorrichtung handeln, durch die Partikel oder Moleküle in die Umgebung abgegeben (delivery device) oder aus der Umgebung aufgenommen (Drainagevorrichtung) oder um einen Stimulator handeln, wobei diese Vorrichtung beispielsweise als entsprechend durchlässiger Behälter ausgebildet ist und die Behälterwände die Oberflächenbereiche 4 erster Art bilden.
**Figur** 1 zeigt als Beispiel des erfindungsgemässen Implantates ein Augmentationselement 31, das zur Erzeugung von zusätzlich zum natürlichen Knochengewebe erwünschtem Knochengewebe, beispielsweise für die Verbreiterung eines Kieferkammes 32 verwendbar ist. Der Kieferkamm 32 und das Augmentationselement 31 sind im Schnitt nach der Implantation dargestellt. Das Augmentationselement 31 weist wiederum einen zentralen Implantatteil 1 auf, der aus einem das Knochenwachstum fördernden Material, beispielsweise aus einem hochporösen Calziumphosphat besteht. In beispielsweise durchgehenden Öffnungen (Innenhohlräume 2') des zentralen Implantatteils 1 sind Stifte des verflüssigbaren Materials angeordnet. Für die Implantation wird das Augmentationselement 31 am entsprechend präparierten Kieferkamm 32 positioniert, derart, dass die Stifte beispielsweise gegen den Kieferkamm 32 gerichtet sind. Dann wird mit einer auf den Stiftquerschnitt abgestimmten Sonotrode 21 Schwingungsenergie in die Stifte eingekoppelt und werden diese gegen den Kieferkamm 32 gepresst. Dadurch wird das verflüssigbare Material mindestens teilweise verflüssigt und in den Kieferkamm und den zentralen Implantatteil gepresst, wodurch das Augmentationselement 31 punktuell am Kieferkamm 32 befestigt ist und der zentrale Implantationsteil (Oberflächenbereiche der ersten Art) mit dem Knochengewebe des Kieferkamms in intensivem Kontakt steht, so dass schon unmittelbar nach der Implantation Zellen aus dem natürlichen Knochen in den zentralen Implantatteil einwandern und knochenbildend wirken können. Das verflüssigbare Material ist im vorliegenden Falle vorteilhafterweise resorbierbar.

## Patentansprüche

1. Augmentationselement zur Implantation in menschlichem oder tierischem Knochengewebe oder durch Knochenersatzmaterial ergänztes Knochengewebe, aufweisend einen zentralen Implantatteil (1) aus einem porösen das Knochenwachstum fördernden Material mit mindestens einer durchgehenden Öffnung und mindestens einen Stift aus einem durch mechanische Schwingungen verflüssigbaren Material, der durch die durchgehenden Öffnungen des zentralen Implantatteils führbar ist, wobei das zentrale Implantatteil in Kontakt mit dem Knochengewebe positionierbar und der mindestens eine in der durchgehenden Öffnung positionierte Stift durch eine auf den Stiftquerschnitt abgestimmte Sonotrode (21), mit welcher Schwingungsenergie in den mindestens einen Stift eingekoppelt wird, gegen das Knochengewebe pressbar ist, wodurch das verflüssigbare Material mindestens teilweise verflüssigt und in das Knochengewebe und den zentralen Implantatteil pressbar ist.

2. Augmentationselement nach Anspruch 1, **dadurch gekennzeichnet, dass** das zentrale Implantationsteil aus hochporösem Calciumphosphat besteht.

3. Augmentationselement nach Anspruch 1 oder 2, **dadurch gekennzeichnet, dass** der zentrale Implantatteil zur Verbreiterung eines menschlichen Kieferkamms ausgerüstet ist.

4. Augmentationselement nach einem der vorangehenden Ansprüche, **dadurch gekennzeichnet, dass** das verflüssigbare Material resorbierbar ist.

5. Augmentationselement nach einem der vorangehenden Ansprüche, **dadurch gekennzeichnet, dass** das mittels mechanischer Schwingungen verflüssigbare Material ein Material mit thermoplastischen Eigenschaften ist.

6. Augmentationselement nach Anspruch 5, **dadurch gekennzeichnet, dass** das mittels mechanischer Schwingungen verflüssigbare Material ein Polymer auf Milch- und/oder Glykolsäurebasis, ein Polyhydroxyalkanoat, ein Polycaprolactone, ein Polysacharid, ein Polypeptid, ein Polydioxanon, ein Polyanhydrid, ein Polyolefin, ein Polyacrylat, ein Polymetacrylat, ein Polycarbonat, ein Polyamid, ein Polyester, ein Polyurethan, ein Polysulfon, ein Polyarylketon, ein Polyimid, ein Polyphenylsulfid, ein Flüssig-Kristall-Polymer, ein Polyacetal, ein halogeniertes Polymer, insbesondere ein halogeniertes Polyolefin, ein Polyphenylensulfid, ein Polysulfon oder ein Polyether ist oder ein Copolymer oder Mischpolymer der genannten Polymere oder ein Verbundwerkstoff, der eines der genannten Polymere enthält, ist.

7. Augmentationselement nach einem der vorangehenden Ansprüche, **dadurch gekennzeichnet, dass** das zentrale Implantatteil eine Mehrzahl von durchgehenden Öffnungen aufweist und das Augmentationselement eine entsprechende Anzahl von durch die durchgehenden Öffnungen führbaren Stiften aufweist.

8. Set enthaltend ein Augmentationselement aufweisend einen zentralen Implantatteil und eine Mehrzahl von Stiften nach einem der Ansprüche 1-7 sowie eine auf den Querschnitt der Stifte abgestimmte Sonotrode.

## Claims

1. An augmentation element for implantation in human or animal bone tissue or in bone tissue supplemented by bone replacement material, comprising a central implant part (1) which is of a porous material promoting bone growth and is with at least one continuous opening and further comprising at least one pin of a material which is liquefiable by way of mechanical oscillations, said pin being able to be led through the continuous opening of the central implant part, wherein the central implant part is positionable in contact with the bone tissue and the at least one pin which is positioned in the continuous opening can be pressed against the bone tissue by way of a sonotrode (21) which is matched to the pin cross section and with which oscillation energy is coupled into the at least one pin, by which means the liquefiable material is at least partly liquefied and can be is pressed into the bone tissue and into the central implant part.

2. An augmentation element according to claim 1, **characterised in that** the central implant part consists of highly porous calcium phosphate.

3. An augmentation element according to claim 1 or 2, **characterised in that** the central implant part is designed for widening a human jaw ridge.

4. An augmentation element according to one of the preceding claims, **characterised in that** the liquifiable material is resorbable.

5. An augmentation element according to one of the preceding claims, **characterised in that** the material which is liquefiable by way of mechanical oscillations is a material with thermoplastic characteristics.

6. An augmentation element according to claim 5, **characterised in that** the material which can be liquefied by way of mechanical oscillations is a polymer based on lactic acid and/or glycolic acid, a polyhydroxyalkanoate, a polycaprolactone, a polysaccharide, a polypeptide, a polydioxanone, a polyanhydride, a polyolefin, a polyacrylate, a polymetacrylate, a polycarbonate, a polyamide, a polyester, a polyurethane, a polysulphone, a polyarylketone, a polyimide, a polyphenyl sulphide, a liquid crystal polymer, a polyacetal, a halogenated polymer, in particular a halogenated polyolefin, a polyphenylene sulphide, a polysulphone or a polyether, or a copolymer or a mixed polymer of the mentioned polymers or a composite material which comprises one of the mentioned polymers.

7. An augmentation implant according to one of the preceding claims, **characterised in that** the central implant part comprises a plurality of continuous openings and the augmentation element comprises a corresponding number of pins which can be led through the continuous openings.

8. A set containing an augmentation element comprising a central implant part and a plurality of pins, according to one of the claims 1-7, as well as a sonotrode which is matched to the cross section of the pins.

## Revendications

1. Élément d'augmentation destiné à l'implantation dans un tissu osseux humain ou animal ou un tissu osseux complété par un matériau de remplacement d'os, comprenant une partie d'implant centrale (1) en un matériaux poreux favorisant la croissance de l'os, comprenant au moins une ouverture continue et au moins une goupille en un matériau liquéfiable par des oscillations mécaniques, qui peut être conduite dans les ouvertures continues de la partie d'implant centrale, la partie d'implant centrale pouvant être positionnée en contact avec le tissu osseux et ladite au moins une goupille positionnée dans l'ouverture continue pouvant être pressée contre le tissu osseux par une sonotrode (21) accordée à la section transversale de la goupille, avec laquelle une énergie d'oscillation est introduite dans ladite au moins une goupille, le matériau liquéfiable étant ainsi au moins partiellement liquéfié et pouvant être pressé dans le tissu osseux et la partie d'implant centrale.

2. Élément d'augmentation selon la revendication 1, **caractérisé en ce que** la partie d'implant centrale est constituée de phosphate de calcium hautement poreux.

3. Élément d'augmentation selon la revendication 1 ou 2, **caractérisé en ce que** la partie d'implant centrale est équipée pour l'élargissement d'une crête alvéolaire humaine.

4. Élément d'augmentation selon l'une quelconque des revendications précédentes, **caractérisé en ce que** le matériau liquéfiable est résorbable.

5. Élément d'augmentation selon l'une quelconque des revendications précédentes, **caractérisé en ce que** le matériau liquéfiable au moyen d'oscillations mécaniques est un matériau ayant des propriétés thermoplastiques.

6. Élément d'augmentation selon la revendication 5, **caractérisé en ce que** le matériau liquéfiable au moyen d'oscillations mécaniques est un polymère à base d'acide lactique et/ou glycolique, un polyhydroxyalcanolate, une polycaprolactone, un polysaccharide, un polypeptide, une polydioxanone, un polyanhydride, une polyoléfine, un polyacrylate, un polyméthacrylate, un polycarbonate, un polyamide, un polyester, un polyuréthane, une polysulfone, une polyarylcétone, un polyimide, un polysulfure de phényle, un polymère cristallin liquide, un polyacétal, un polymère halogéné, notamment une polyoléfine halogénée, un polysulfure de phénylène, une polysulfone ou un polyéther, ou un copolymère ou polymère mixte des polymères mentionnés ou un matériau composite qui contient un des polymères mentionnés.

7. Élément d'augmentation selon l'une quelconque des revendications précédentes, **caractérisé en ce que** la partie d'implant centrale comprend une pluralité d'ouvertures continues et l'élément d'augmentation comprend un nombre correspondant de goupilles pouvant être conduites dans les ouvertures continues.

8. Ensemble contenant un élément d'augmentation comprenant une partie d'implant centrale et une pluralité de goupilles selon l'une quelconque des revendications 1 à 7, ainsi qu'une sonotrode accordée à la section transversale des goupilles.
